# EUROPEAN PATENT APPLICATION

(11) **EP 4 649 967 A1**
(43) Date of publication of application: **19.11.2025**
(21) Application number: 23926515.0
(22) Date of filing: 07.09.2023
(51) Int. Cl.: A61L 2/00, A61L 2/14

(54) **ELECTRONIC DEODORANT DEVICE USING PLASMA**

(30) Priority: 09.03.2023 KR 20230031355; 11.05.2023 KR 20230060831
(71) Applicant: Codesteri Inc., Seoul 04763 (KR); IUCF-HYU (Industry-University Cooperation Foundation Hanyang University), Seoul 04763 (KR)
(72) Inventor: LIM, Tae Ho, Seoul 06276 (KR); CHOI, Jong Bong, Seoul 04761 (KR); JANG, Yong Woo, Guri-si Gyeonggi-do 11901 (KR); SEO, Jung Chi, Seoul 04795 (KR); OH, Yong Hak, Suwon-si Gyeonggi-do 16398 (KR)
(74) Representative: Dr. Gassner & Partner mbB
(86) International application number: PCT/KR2023/013462
(87) International publication number: WO 2024/185942

(57) **Abstract**

An electronic deodorant device is disclosed. The electronic deodorant device comprises: a housing; a nozzle head that is provided in the housing and has a first flow path and a second flow path; a gas circulation line that circulates the gas introduced through the first flow path within the housing and supplies same to the second flow path; an air pump that is installed on the gas circulation line and introduces external gas through the first flow path; and a plasma generation part that is installed in the circulating gas line in a section between the air pump and the second flow path and performs plasma discharge treatment on the gas.

## Description

### [Technical Field]

The present invention relates to an electronic deodorant device using plasma, and more particularly, to an electronic deodorant device using plasma, capable of sterilizing bacteria that cause an odor on the skin of a human or an animal, such as bromhidrosis.

### [Background Art]

Bromhidrosis (body odor) is an odor caused by fatty acid organic matter secreted through sweat and bacteria, and chemical compound deodorants are currently used to remove the odor for cosmetic and etiquette purposes.

However, controversy of deodorants for suppressing bromhidrosis over harmfulness to a human body is increasing. In particular, harmfulness of chemical ingredients contained in the deodorants to suppress sweat secretion by blocking pores is being raised, which may cause breast cancer, deterioration of thyroid function (triclosan), sweat secretion hormone disorder, skin damage, and pigmentation due to closed sweat glands (aluminum salt) in addition to haplodermatitis. Accordingly, an odor removal technology capable of suppressing bromhidrosis without harmfulness of chemical ingredients is required.

### [Disclosure]

### [Technical Problem]

The present invention provides an electronic deodorant device using plasma, capable of sterilizing bacteria that cause bromhidrosis by supplying the plasma.

In addition, the present invention provides an electronic deodorant device using plasma, capable of stably maintaining an activation state of the plasma and supplying the plasma at a high concentration.

### [Technical Solution]

According to an embodiment of the present invention, an electronic deodorant device includes: a housing; a nozzle head provided in the housing, and having a first flow path and a second flow path; a gas circulation line configured to circulate a gas introduced through the first flow path within the housing, and supply the gas to the second flow path; an air pump installed on the gas circulation line, and configured to introduce an external gas through the first flow path; and a plasma generation part installed on the circulation gas line in a section between the air pump and the second flow path, and configured to perform plasma discharge treatment on the gas.

In addition, the housing may have a cylindrical shape in which a partial region of an outer circumferential surface is concavely recessed inward, and the nozzle head may be located in the partial region.

In addition, the nozzle head may include: a body having an inner space with an open front surface; and a cover connected to the front surface of the body, and having a suction port and a discharge port, the first flow path may communicate with the inner space of the body, and the second flow path may be connected to the discharge port.

In addition, the electronic deodorant device may further include a sealing formed of a silicone material and provided along a perimeter of the partial region.

In addition, the nozzle head may include: a body having an inner space with an open front surface; and a separation plate configured to partition the inner space of the body into a first space and a second space, and having a suction port connected to the first flow path and a discharge port connected to the second flow path, and the first flow path may connect one end of the gas circulation line and the first space, while the second flow path may connect an opposite end of the gas circulation line and the discharge port.

In addition, the discharge port may be formed at a center of the separation plate, and a plurality of suction ports may be formed at a periphery of the discharge port.

In addition, the second space may have a diameter that is gradually increased from the separation plate toward the open front surface of the body.

In addition, the plasma generation part may include: a first electrode formed of a conductive material, and having a tube shape; a dielectric tube inserted into the first electrode tube, formed of a dielectric material, and having a tube shape; a second electrode formed of a conductive material, having a tube shape, and partially inserted into the dielectric tube; and a power source configured to apply a voltage to the first electrode and the second electrode.

In addition, the first electrode, the dielectric tube, and the second electrode may overlap each other in partial regions.

In addition, the plasma generation part may further include: a plasma generation body formed of a dielectric material, and configured to provide a space in which the first electrode, the dielectric tube, and the second electrode are located within the plasma generation body, and the space formed in the plasma generation body may include: a flow path decrease section in which one end of the dielectric tube is located, and having a flow path width that is gradually decreased as the flow path decrease section gets farther away from the one end of the dielectric tube; and a flow path increase section connected to the flow path decrease section, and having a flow path width that is gradually increased as the flow path increase section gets farther away from the one end of the dielectric tube.

In addition, a plurality of protrusions protruding forward to a predetermined length may be formed on a front surface of the nozzle head.

### [Advantageous Effects]

According to the present invention, an electronic deodorant device can sterilize bacteria that cause bromhidrosis by supplying plasma to the skin of a human or an animal.

In addition, according to the present invention, the electronic deodorant device can supply the plasma at a high concentration by activating a gas into a plasma state, and recirculating and reactivating the activated gas.

### [Description of Drawings]

FIG. 1 is a perspective view showing an electronic deodorant device according to one embodiment of the present invention.
FIG. 2 is a view showing an inside of a housing of FIG. 1.
FIG. 3 is a sectional view showing a nozzle head of FIG. 1.
FIG. 4 is a sectional view showing a plasma generation part of FIG. 1.
FIG. 5 is a view showing a state of using the electronic deodorant device according to the embodiment of the present invention.
FIG. 6 is a view showing a state in which the electronic deodorant device and the skin of a user make close contact with each other.
FIG. 7 is a perspective view showing an electronic deodorant device according to another embodiment of the present invention.
FIG. 8 is a perspective view showing an inside of a housing of FIG. 7.
FIG. 9 is a sectional view showing a nozzle head of FIG. 7.
FIG. 10 is a view showing a state of using the electronic deodorant device according to the embodiment of FIG. 7.
FIG. 11 is a sectional view showing a nozzle head according to another embodiment of the present invention.
FIG. 12 is a view showing a state of using the electronic deodorant device according to another embodiment of the present invention.

### [Best Mode]

According to an embodiment of the present invention, an electronic deodorant device includes: a housing; a nozzle head provided in the housing, and having a first flow path and a second flow path; a gas circulation line configured to circulate a gas introduced through the first flow path within the housing, and supply the gas to the second flow path; an air pump installed on the gas circulation line, and configured to introduce an external gas through the first flow path; and a plasma generation part installed on the circulation gas line in a section between the air pump and the second flow path, and configured to perform plasma discharge treatment on the gas.

### [Mode for Invention]

Hereinafter, exemplary embodiments of the present invention will be described in detail with reference to the accompanying drawings. However, the technical idea of the present invention is not limited to the embodiments described herein, but may be embodied in different forms. The embodiments introduced herein are provided to sufficiently deliver the idea of the present invention to those skilled in the art so that the disclosed contents may become thorough and complete.

When it is mentioned in the present disclosure that one element is on another element, it means that one element may be directly formed on another element, or a third element may be interposed between one element and another element. Further, in the drawings, thicknesses of films and regions are exaggerated for effective description of the technical contents.

In addition, although the terms such as first, second, and third have been used to describe various elements in various embodiments of the present disclosure, the elements are not limited by the terms. The terms are used only to distinguish one element from another element. Therefore, an element mentioned as a first element in one embodiment may be mentioned as a second element in another embodiment. The embodiments described and illustrated herein include their complementary embodiments, respectively. Further, the term "and/or" used in the present disclosure is used to include at least one of the elements enumerated before and after the term.

As used herein, an expression in a singular form includes a meaning of a plural form unless the context clearly indicates otherwise. Further, the terms such as "including" and "having" are intended to designate the presence of features, numbers, steps, elements, or combinations thereof described herein, and shall not be construed to preclude any possibility of the presence or addition of one or more other features, numbers, steps, elements, or combinations thereof. In addition, the term "connection" used herein is used to include both indirect and direct connections of a plurality of elements.

Further, in the following description of the present invention, detailed descriptions of known functions or configurations incorporated herein will be omitted when they may make the gist of the present invention unnecessarily unclear.

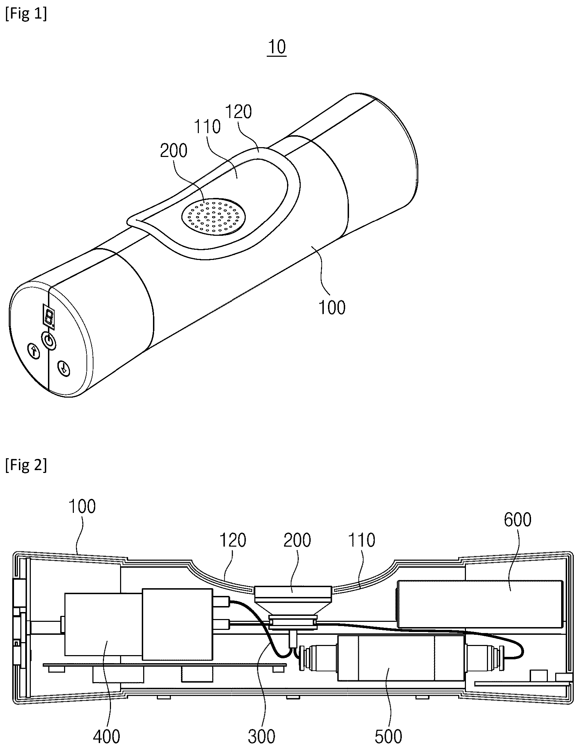

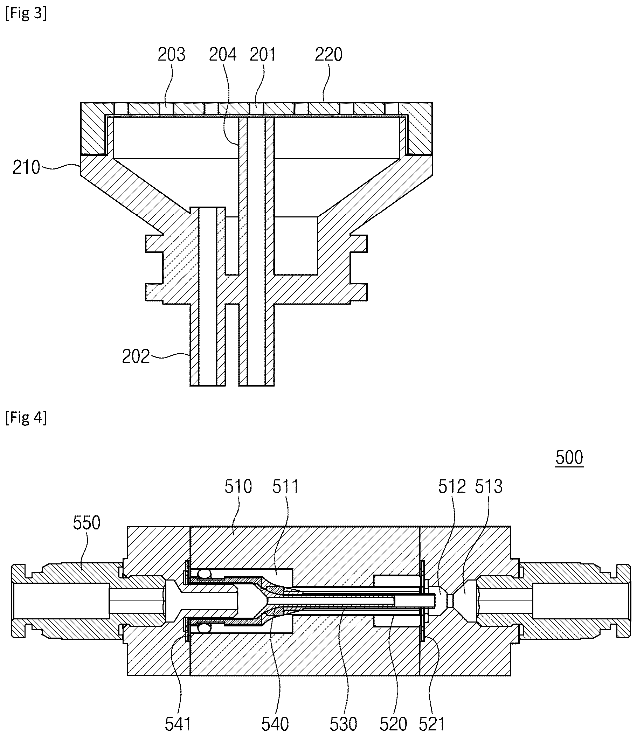

FIG. 1 is a perspective view showing an electronic deodorant device according to one embodiment of the present invention, FIG. 2 is a view showing an inside of a housing of FIG. 1, FIG. 3 is a sectional view showing a nozzle head of FIG. 1, and FIG. 4 is a sectional view showing a plasma generation part of FIG. 1.

Referring to FIGS. 1 to 4, an electronic deodorant device 10 may generate plasma, and supply the generated plasma so as to sterilize bacteria. According to one example, the electronic deodorant device 10 may supply the plasma to remove the bacteria that cause bromhidrosis, which causes an odor on the skin of a human or an animal. For example, an odor may be generated by fatty acid organic matter secreted from sweat and skin bacteria, and the electronic deodorant device 10 may supply the plasma to remove the odor.

The electronic deodorant device 10 may include a housing 100, a nozzle head 200, a gas circulation line 300, an air pump 400, and a plasma generation part 500.

The housing 100 may have a cylindrical shape, and may have a space formed therein. The housing 100 may be configured such that a partial region 110 of an outer circumferential surface may be concavely recessed inward. A sealing 120 may be provided along a perimeter of the partial region 110. The sealing 120 may have a ring shape, and may improve contact capability with the skin. The sealing 120 may be formed of a material that is friendly to a human body. According to an embodiment, the sealing 120 may be formed of a silicone material.

A nozzle head 200 may be provided in the partial region 110 of the housing 100. The nozzle head 200 may suck external air, and discharge plasma to an outside. The nozzle head 200 may include a first flow path 202, a second flow path 204, a body 210, and a cover 220.

The body 210 may have a funnel shape having a predetermined diameter. In detail, the body 210 may have a space formed therein with an open front surface, and may have a diameter that is gradually decreased toward a rear surface.

The cover 220 may be coupled to the open front surface of the body 210, and may have a discharge port 201 and a suction port 203. The discharge port 201 may be formed in a central region of the cover 220, and a plurality of suction ports 203 may be formed at a periphery of the discharge port 201. The suction ports 203 may be formed along the periphery of the discharge port 201 at uniform intervals. According to an embodiment, the suction port 203 may have the same diameter as the discharge port 201. Alternatively, the suction port 203 may have a diameter that is smaller than a diameter of the discharge port 201.

The first flow path 202 may be provided inside the body 210, and may have an upper end located inside the body 210. The first flow path 202 may be connected to the suction port 203 through an inner space of the body 210.

The second flow path 204 may be provided inside the body 210, and may have an upper end connected to the discharge port 201.

The gas circulation line 300 may be provided inside the housing 100. The gas circulation line 300 may have a predetermined length, and may provide a flow path through which a gas may circulate. One end of the gas circulation line 300 may be connected to the first flow path 202, and an opposite end of the gas circulation line 300 may be connected to the second flow path 204. The gas introduced through the suction port 203 and the first flow path 202 may circulate along the gas circulation line 300, and may be discharged to a front side of the nozzle head 200 through the second flow path 204 and the discharge port 201.

The air pump 400 may be installed on the gas circulation line 300, and may generate power that allows the gas to circulate along the gas circulation line 300.

The plasma generation part 500 may be installed on the gas circulation line 300 between the air pump 400 and the second flow path 204. The plasma generation part 500 may perform plasma discharge treatment on the gas circulating along the gas circulation line 300 so as to generate plasma. The gas that has been subjected to the plasma discharge treatment may include ozone, nitrogen oxide, hydroxide base, carbon dioxide, and carbon monoxide. The ozone, the nitrogen oxide, the hydroxide base, and the like may be in a radical state.

The plasma generation part 500 may include a plasma generation body 510, a first electrode 520, a dielectric tube 530, a second electrode 540, and a power source (not shown).

The plasma generation body 510 may be located inside the housing 100, and may have a predetermined shape. According to an embodiment, the plasma generation body 510 may have a cylindrical shape, and may have a space 511 formed therein. The first electrode 520, the dielectric tube 530, and the second electrode 540 may be located in the inner space 511. The plasma generation body 510 may be formed of an insulating material.

The first electrode 520 may be formed of a conductive material, and may have a cylindrical shape having a predetermined length. The first electrode 520 may have a first connection terminal 521, and may be connected to the power source through the first connection terminal 521.

The dielectric tube 530 may be formed of a dielectric material, and may have a cylindrical shape having a predetermined length. The dielectric tube 530 may have at least a partial region inserted into the first electrode 520, and may have a front end protruding forward of the first electrode 520.

The second electrode 540 may be formed of a conductive material, and may have a cylindrical shape having a predetermined length. The second electrode 540 may have a partial region having a diameter that is smaller than a diameter of the dielectric tube 530, and inserted into the dielectric tube 530. A front end of the second electrode 540 may be located on an inner side of the first electrode 520. As a result, the first electrode 520, the dielectric tube 530, and the second electrode 540 may overlap each other in at least partial regions. The second electrode 540 may have a second connection terminal 541, and may be connected to a battery 600 through the second connection terminal 541.

A space located on a front side of the first electrode 520 in the inner space of the plasma body 510 may include a flow path decrease section 512 and a flow path increase section 513. The flow path decrease section 512 may be a section in which the front end of the dielectric tube 530 is located, and may have a flow path width that is gradually decreased as the flow path decrease section 512 gets farther away from the front end of the dielectric tube 530. The flow path increase section 513 may be connected to the flow path decrease section 512, and may have a flow path width that is gradually increased as the flow path increase section 513 gets farther away from the front end of the dielectric tube 530.

The gas circulating through the gas circulation line 300 may be introduced into the plasma generation body 510 through a connection member 550 provided at a rear end of the plasma generation body 510, and may be introduced into the flow path decrease 512 section through an inner flow path of the second electrode 540 and an inner flow path of the dielectric tube 530. In this process, an electric field may be formed on an inner side of the dielectric tube 530 due to a difference in voltages applied to the first electrode 520 and the second electrode 540, and the gas may be activated by the electric field. As the activated gas sequentially passes through the flow path decrease section 512 and the flow path increase section 513, a pressure of the activated gas may be decreased, and a flow rate of the activated gas may be increased. The activated gas with the increased flow rate may be rapidly introduced into the second flow path 204 along the gas circulation line 300, and may be supplied to the front side of the nozzle head 200 through the discharge port 201.

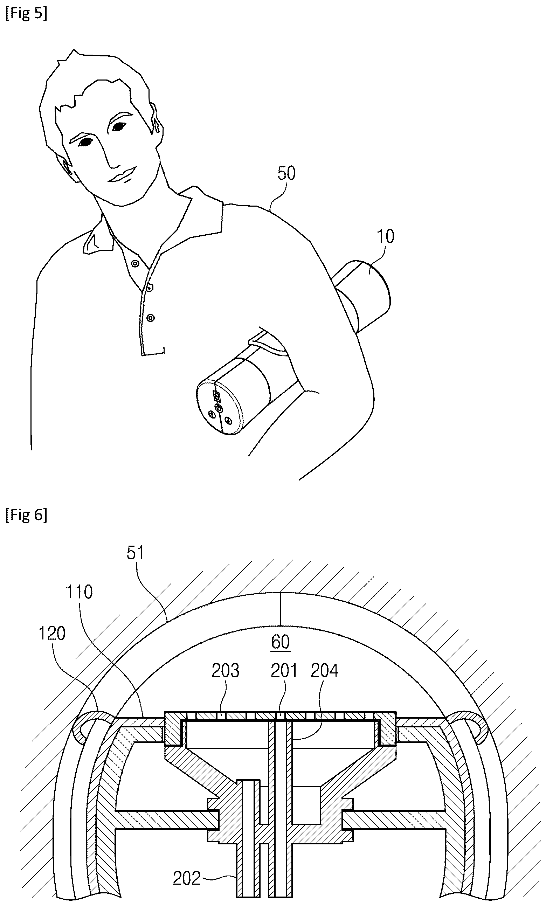

FIG. 5 is a view showing a state of using the electronic deodorant device according to the embodiment of the present invention, and FIG. 6 is a view showing a state in which the electronic deodorant device and the skin of a user make close contact with each other.

Referring to FIGS. 5 and 6, a user 50 may supply plasma to eliminate an odor occurring in an armpit. The user 50 may fit the electronic deodorant device 10 in the armpit such that the partial region 110 may face skin 51. In this case, the sealing 120 may make close contact with the skin of the user 50, so that the partial region 110 of the housing 100 and the skin 51 of the user may form a sealed space 60.

When the air pump 400 is driven by an operation of the deodorant device 10, a gas remaining in the sealed space 60 between the partial region 110 and the skin 30 may be introduced into the first flow path 202 through the suction port 203. The gas may be air. The gas may circulate along the gas circulation line 300, and may be introduced into the plasma generation body 510. The gas may be introduced into the flow path decrease section 512 through the inner flow path of the second electrode 540 and the inner flow path of the dielectric tube 530, and may be activated by the electric field formed on the inner side of the dielectric tube 530 during this process. As the activated gas sequentially passes through the flow path decrease section 512 and the flow path increase section 513, a flow rate of the activated gas may be increased. The activated gas may be introduced into the second path 204 along the gas circulation line 300, and may be supplied to the sealed space 60 through the discharge port 201. The activated gas may be supplied to the skin 51 to sterilize bacteria. The activated gas may remain within the sealed space 60, and may be introduced back into the gas circulation line 300 through the suction port 203. The activated gas may be reactivated while passing through the plasma treatment part 500, and may be resupplied to the skin 51 through the nozzle head 200. As described above, the activated gas may be recirculated and reactivated in the sealed space 60. Due to the reactivation, energy of the activated gas may be increased, and a radical state may be stably maintained. This may enhance a bacteria sterilization effect.

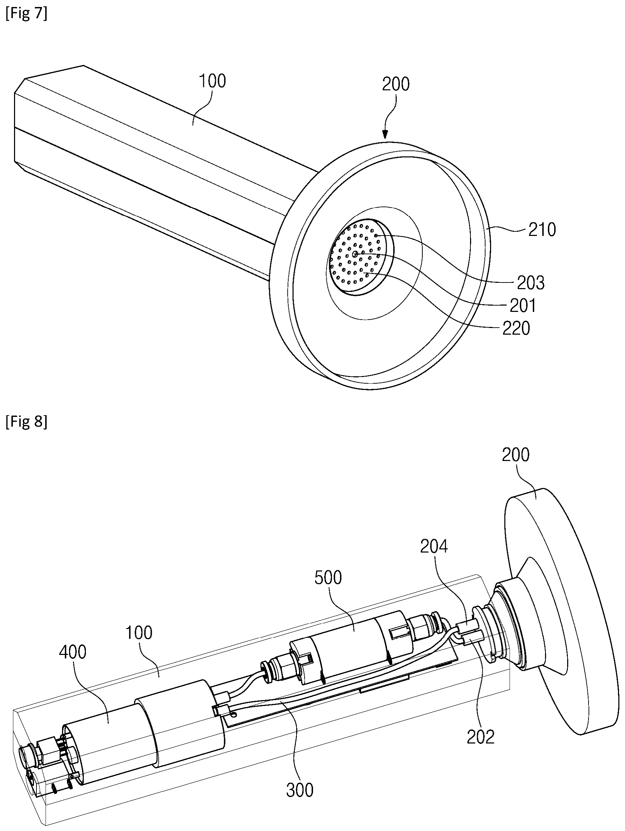

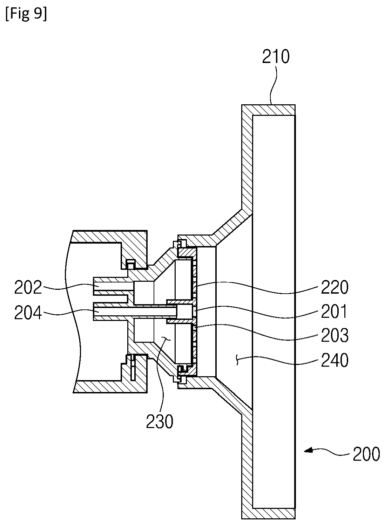

FIG. 7 is a perspective view showing an electronic deodorant device according to another embodiment of the present invention, FIG. 8 is a perspective view showing an inside of a housing of FIG. 7, and FIG. 9 is a sectional view showing a nozzle head of FIG. 7.

Referring to FIGS. 7 to 9, an electronic deodorant device 10 may include a housing 100, a nozzle head 200, a gas circulation line 300, an air pump 400, and a plasma generation part 500.

The housing 100 may have a predetermined shape and a predetermined size, and may have a space formed therein. According to an embodiment, the housing 100 may have a hexahedral shape having a predetermined length.

The nozzle head 200 may be provided at a front end of the housing 100, and may be detachable attached to the housing 100. The nozzle head 200 may have a first flow path 202 and a second flow path 204. According to an embodiment, the first flow path 202 and the second flow path 204 may be located in a central region of the nozzle head 200.

The nozzle head 200 may include a body 210 and a separation plate 220.

The body 210 may have a funnel shape. In detail, the body 210 may have a space formed therein with an open front surface, and may have a diameter that is gradually decreased toward a rear surface. The front surface of the body 210 may have a diameter that is larger than a cross section of the housing 100.

When the separation plate 220 is located on an inner side of the body 210, the separation plate 220 may partition an inner space of the body 210 into a first space 230 and a second space 240. Based on the separation plate 220, the first space 230 may be located adjacent to the housing 100, and the second space 240 may be located on an opposite side of the first space 230.

A discharge port 201 may be formed in a center region of the separation plate 220, and a plurality of suction ports 203 may be formed at a periphery of the discharge port 201. The suction ports 203 may be formed along the periphery of the discharge port 201 at uniform intervals. According to an embodiment, the suction port 203 may have a diameter that is smaller than a diameter of the discharge port 201. The discharge port 201 may communicate with the second flow path 204, and the suction port 203 may communicate with the first flow path 202 through the first space 230.

The gas circulation line 300, the air pump 400, and the plasma generation part 500 may have configurations that are identical to the configurations described in FIGS. 1 to 4, so that detailed descriptions thereof will be omitted.

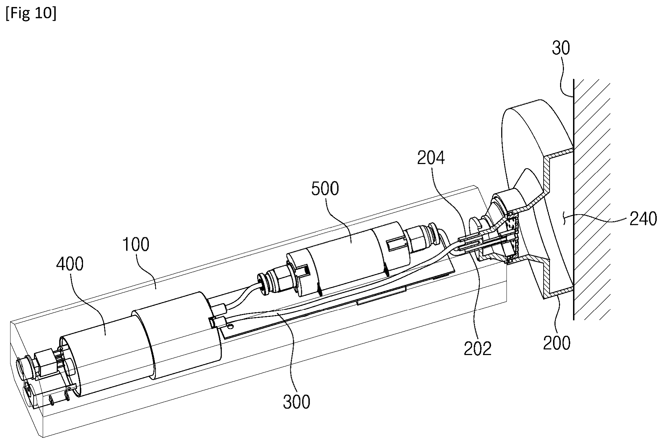

FIG. 10 is a view showing a state of using the electronic deodorant device according to the embodiment of FIG. 7.

Referring to FIG. 10, a user may operate the electronic deodorant device 10 while the nozzle head 200 makes close contact with skin 30. When the air pump 400 is driven, a gas remaining in the second space 240 between the nozzle head 200 and the skin 30 may be introduced into the first flow path 202 through the suction port 203. The gas may be air. The gas may circulate along the gas circulation line 300, and may be introduced into the plasma generation body 510. The gas may be introduced into the flow path decrease section 512 through the inner flow path of the second electrode 540 and the inner flow path of the dielectric tube 530, and may be activated by the electric field formed on the inner side of the dielectric tube 530 during this process. As the activated gas sequentially passes through the flow path decrease section 512 and the flow path increase section 513, a flow rate of the activated gas may be increased. The activated gas may be introduced into the second path 204 along the gas circulation line 300, and may be supplied to the front side of the nozzle head 200 through the discharge port 201. The activated gas may be supplied to the skin to sterilize bacteria. The activated gas may remain within the space 240 sealed by the nozzle head 200 and the skin 30, and may be introduced back into the gas circulation line 300 through the suction port 203. The activated gas may be reactivated while passing through the plasma treatment part 500, and may be resupplied to the skin 30 through the nozzle head 200. As described above, the space 240 in which the activated gas is supplied may be sealed by the nozzle head 200 and the skin 30, the activated gas may be recirculated and reactivated. Due to the reactivation, energy of the activated gas may be increased, and a radical state may be stably maintained. This may enhance a bacteria sterilization effect.

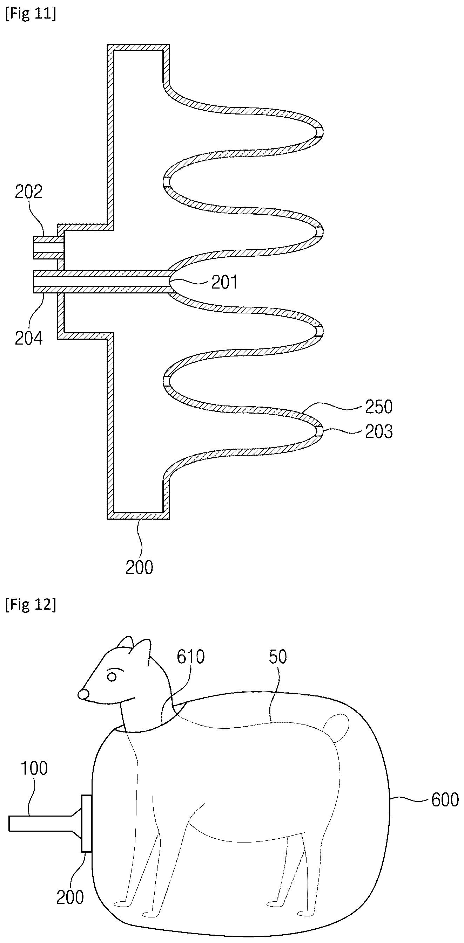

FIG. 11 is a sectional view showing a nozzle head according to another embodiment of the present invention.

Referring to FIG. 11, a plurality of protrusions 250 may be formed on a front surface of a nozzle head 200. The protrusions 250 may protrude forward of the nozzle head 200 to a predetermined length, and may have diameters that are gradually decreased toward ends thereof. A discharge port 201 may be formed at the end of the protrusion 250.

The nozzle head 200 having the structure described above may be configured such that the protrusions 250 may function as brushes, and the discharge port 203 may supply an activated gas to the skin as closely as possible. The nozzle head 200 may directly supply plasma to the scalp while the protrusions 250 comb hair of a human. In addition, in a case of an animal having a great amount of hair, such as a dog and a cat, the nozzle head 200 may directly supply the plasma to the skin while combing the hair.

FIG. 12 is a view showing a state of using the electronic deodorant device according to another embodiment of the present invention.

Referring to FIG. 12, the electronic deodorant device 10 may further include a protective cover 600. The protective cover 600 may have a space in which a body of an animal 50 may enter formed therein, and may have one region having an opening 610 through which a head of the animal 50 may come out. The protective cover 600 may be formed of a material capable of blocking entry/exit of air. According to one example, the protective cover 600 may be formed of a synthetic resin film having a thin thickness.

The protective cover 600 may be coupled to the nozzle head 200. The air inside the protective cover 600 may be introduced into the gas circulation line 300 through the suction port 203, activated in the plasma treatment part 500, and supplied into the protective cover 600 through the discharge port 201. The air inside the protective cover 600 may be reactivated and resupplied into the protective cover 600 while repeatedly circulating along the gas circulation line 300 through the process described above. As this process is repeatedly performed, a concentration of the activated gas inside the protective cover 600 may be gradually increased. As the inside of the protective cover 600 is replaced with the activated gas, bacteria attached to the skin or hair of the animal 50 may be sterilized. Since the activated gas having a high concentration may be supplied to a skin surface of the animal 50, the bacteria attached to the skin of the animal 50 may be effectively sterilized.

Although the exemplary embodiments of the present invention have been described in detail above, the scope of the present invention is not limited to a specific embodiment, and shall be interpreted by the appended claims. In addition, it is to be understood by a person having ordinary skill in the art that various changes and modifications can be made without departing from the scope of the present invention.

### [Industrial Applicability]

An electronic deodorant device according to an embodiment of the present invention may be used to sterilize bacteria that cause an odor on the skin of a human or an animal, such as bromhidrosis.

## Claims

1. An electronic deodorant device comprising:
a housing;
a nozzle head provided in the housing, and having a first flow path and a second flow path;
a gas circulation line configured to circulate a gas introduced through the first flow path within the housing, and supply the gas to the second flow path;
an air pump installed on the gas circulation line, and configured to introduce an external gas through the first flow path; and
a plasma generation part installed on the circulation gas line in a section between the air pump and the second flow path, and configured to perform plasma discharge treatment on the gas.

2. The electronic deodorant device of claim 1, wherein the housing has a cylindrical shape in which a partial region of an outer circumferential surface is concavely recessed inward, and
the nozzle head is located in the partial region.

3. The electronic deodorant device of claim 2, wherein the nozzle head includes:
a body having an inner space with an open front surface; and
a cover connected to the front surface of the body, and having a suction port and a discharge port,
the first flow path communicates with the inner space of the body, and
the second flow path is connected to the discharge port.

4. The electronic deodorant device of claim 2, further comprising a sealing formed of a silicone material and provided along a perimeter of the partial region.

5. The electronic deodorant device of claim 1, wherein the nozzle head includes:
a body having an inner space with an open front surface;
and
a separation plate configured to partition the inner space of the body into a first space and a second space, and having a suction port connected to the first flow path and a discharge port connected to the second flow path, and
the first flow path connects one end of the gas circulation line and the first space, while the second flow path connects an opposite end of the gas circulation line and the discharge port.

6. The electronic deodorant device of claim 5, wherein the discharge port is formed at a center of the separation plate, and
a plurality of suction ports are formed at a periphery of the discharge port.

7. The electronic deodorant device of claim 5, wherein the second space has a diameter that is gradually increased from the separation plate toward the open front surface of the body.

8. The electronic deodorant device of claim 1, wherein the plasma generation part includes:
a first electrode formed of a conductive material, and having a tube shape;
a dielectric tube inserted into the first electrode tube, formed of a dielectric material, and having a tube shape;
a second electrode formed of a conductive material, having a tube shape, and partially inserted into the dielectric tube; and
a power source configured to apply a voltage to the first electrode and the second electrode.

9. The electronic deodorant device of claim 8, wherein the first electrode, the dielectric tube, and the second electrode overlap each other in partial regions.

10. The electronic deodorant device of claim 8, wherein the plasma generation part further includes:
a plasma generation body formed of a dielectric material, and configured to provide a space in which the first electrode, the dielectric tube, and the second electrode are located within the plasma generation body, and
the space formed in the plasma generation body includes:
a flow path decrease section in which one end of the dielectric tube is located, and having a flow path width that is gradually decreased as the flow path decrease section gets farther away from the one end of the dielectric tube; and
a flow path increase section connected to the flow path decrease section, and having a flow path width that is gradually increased as the flow path increase section gets farther away from the one end of the dielectric tube.

11. The electronic deodorant device of claim 1, wherein a plurality of protrusions protruding forward to a predetermined length are formed on a front surface of the nozzle head.
